# EUROPEAN PATENT SPECIFICATION

(11) **EP 1 328 192 B1**
(45) Date of publication and mention of the grant of the patent: **05.01.2011**
(21) Application number: 02713061.6
(22) Date of filing: 02.04.2002
(51) Int. Cl.: A61B 5/00

(54) **INTEGRATED SAMPLE TESTING METER**
INTEGRIERTES BLUTPROBEMESSGERÄT MIT TESTSTREIFEN
APPAREIL D'ANALYSE D'ECHANTILLON INTEGRE

(30) Priority: 29.03.2001 US 280321 P
(43) Date of publication of application: 23.07.2003
(73) Proprietor: Lifescan Scotland Ltd, Inverness, IV2 3ED, Scotland (GB)
(72) Inventor: MOERMAN, Piet, B-9831 St. Martens-Latem (BE); SHAANAN, Gad, Suite E2, San Diego, CA 92121 (US); MAINVILLE, Patrick, Montreal, Quebec H2C 2J9 (CA); ORBAN, Benoit, St. Lambert, Quebec J4P 2S6 (CA); COLEY, Benjamin, Laval, Quebec H7M 5C6 (CA); FRANCOVICH, Walter, Pierrefonds, Quebec H9J 3S8 (CA); BODE, Andreas, D-10961 Berlin (DE); STIENE, Matthias, Inverness IV2 3QG (GB); GRIFFITH, Alun, Inverness IV2 3XQ (GB)
(74) Representative: Mercer, Christopher Paul
(86) International application number: PCT/GB2002/001599
(87) International publication number: WO 2002/078533

(56) References cited:
- EP-A- 0 931 507
- WO-A-99/59657
- DE-A- 19 819 407
- US-A- 6 093 156
- PATENT ABSTRACTS OF JAPAN vol. 2000, no. 07, 29 September 2000 (2000-09-29) & JP 2000 116626 A (KDK CORP), 25 April 2000 (2000-04-25)

## Description

### Field of the Invention

The present invention relates to an integrated sample testing meter for use in sampling and analyzing analytes, particularly glucose, in fluids such as blood or interstitial fluid.

### Background of the Invention

Glucose monitoring is a fact of everyday life for diabetic individuals. The accuracy of such monitoring can literally mean the difference between life and death. Generally, a diabetic patient measures blood glucose levels several times a day to monitor and control blood sugar levels. Failure to test blood glucose levels accurately and on a regular basis can result in serious diabetes-related complications, including cardiovascular disease, kidney disease, nerve damage and blindness. A number of glucose meters are currently available which permit an individual to test the glucose level in a small sample of blood.

Many of the glucose meter designs currently available make use of a disposable test strip which, in combination with the meter, electrochemically or photometrically measures the amount of glucose in the blood sample. To use these meters, the user first punctures a finger or other body part using a lancet to produce a small sample of blood or interstitial fluid. The sample is then transferred to a disposable test strip. The inconvenience of taking several measurements a day, as well as the pain inflicted by currently available lancets, often discourage disciplined and frequent testing.

While the fingertip is generally used for sampling blood, due to the rich capillary bed of the skin of the fingertip, the fingertip is also particularly sensitive to pain, due to a rich supply of pain receptors in the finger tip as well. When a puncture is too deep, too close to a recent puncture or not deep enough and requires an additional puncture, the pain increases significantly. Pain may also be increased if the lancet penetrates slowly or is withdrawn slowly. Furthermore, the user may be forced to make a larger puncture than is necessary to form a sufficient amount of blood, due to losses during the transfer between the puncture site and the test strip.

The process of measuring blood glucose levels requires several steps and several different accessories, including a lancing device, a lancet, a supply of test strips and a glucose meter. Each accessory has a different function. The user must have a flat surface available to unpack and lay down the accessories within easy reach. This, by itself, poses a challenge for those who need to take measurements while participating in outdoor activities. Flat surfaces are often not available and this can discourage a person from taking a measurement. This can be disadvantageous because blood glucose levels may change significantly during an outdoor activity.

Even if a user can find a flat surface, the user has to carry out the following steps. The user: charges the lancing device with a fresh lancet; opens a vial of strips; removes a strip; inserts the strip into the meter; re-closes the vial; checks for the correct calibration code on the meter; picks up the lancing device; lances the skin of the finger or other body part; lays down the lancing device; squeezes or massages the finger to yield an adequate blood sample; transfers the sample to the test strip for analysis; waits for the meter to analyze the sample; removes the strip from the test meter; discards the strip; and finally re-packs all of the accessories. As set forth above, the standard procedure for taking a glucose measurement requires the use of multiple, separate components and the execution of a number of steps requiring manual user intervention.

Generally, the user is required to transfer a small volume of sample to a sample-receiving area on the test strip. Generally, test strips are quite small and the sample-recciving area is therefore even smaller. This transfer step is a difficult task for many users. Moreover, there has recently been a trend towards the use of test strips requiring ever smaller amounts of sample. (This allows the use of smaller punctures and therefore less painful lancing.) However, the use of smaller samples increases the difficulty in transferring the sample to the sample-receiving area on the test strip. This is especially difficult for users with poor eyesight, a common complication for diabetics.

The pain, inconvenience, cost, slowness, complexity and discreteness of taking a blood glucose measurement are barriers to the frequent monitoring of glucose levels.

Patients often do not comply with doctor recommendations to frequently test glucose levels due to the numerous obstacles involved. DE 19819401 discloses a testing meter with cartridge and automatic mechanism to bring the strip in the measuring position.

It is an aim of the present invention to provide, at least in part, a solution to the above problems.

### Summary of the Invention

Accordingly, the present invention provides an integrated sample-testing meter comprising a single modular housing as defined in appended claim 1. Further preferred features of the invention are defined in subclaims 2-10.

Additional features of the invention and related aspects of the invention are outlined as follows:

The meter of the present invention preferably includes electrical or electronic circuitry for controlling its operation. Such circuitry may be hard-wired or may comprise a microcomputer or similar device. Such circuitry will in particular include all the components of the sensor and will be arranged to carry out the analysis of the sample.

Preferably, the circuitry also includes a visual display unit from which the user can read out the results of any particular test. The display may also be adapted to provide a display of the data, as explained in more detail below.

Preferably, the circuitry includes means, such as a touch sensitive display, a microphone and voice activated software or control buttons, for entering data into the meter.

Preferably, the meter is arranged such that, in use, when strip is in the sample-receiving position, its sample receiving area is spaced from the puncture site by a distance of from about 0.4 mm to about 1.3 mm and preferably by a distance of about 0.7 mm to about 0.9 mm.

The pressure device may comprise a pump adapted to apply a negative pressure to a volume in the meter having an aperture for location on the skin of the user.

Preferably, however, the pressure device comprises a pressure ring arranged to be located, in use, on the user's skin and to apply pressure at the edges of the ring to increase the amount of fluid available at the centre of the ring.

The pressure ring may be shaped to conform to the shape of the area of the skin to which it is to be applied. For instance, if the meter is intended for use on the forearm, the pressure ring will be generally planar. However, if the meter is intended for use on a finger, the pressure ring will be curved.

Preferably, the pressure ring has a multi-contoured surface to increase the pressure gradient from the outside to the inside of the ring.

Advantageously, the pressure ring is part of a lancet cap which covers the lancet in its retracted position. Preferably, the lancet cap includes a means, such as a side wall, which co-operates with the lancet drive train to ensure that the lancet travels along approximately the same path on each activation of the drive train.

The lancet cap may be an integral part of the housing. Preferably, however, the lancet cap is detachably mounted on the housing. This may be achieved by use of screwthread or bayonet type fixings or by use .of a snap fit connection.

If desired, the meter can include at least two interchangeable lancet caps, for instance a lancet cap for forearm use and a lancet cap for finger use.

The lancet may be any of the types of lancet at present in use in the art. The term "lancet" includes finger-sticking devices of the type known in the art. Preferably, the lancet is removably attached to the lancet drive train so that the lancet can be disposed of after one or several uses.

Preferably, the lancet drive train is spring driven. Alternatively, the lancet drive train is driven electromagnetically. The drive train is arranged such that, on actuation, the lancet moves to the extended position and is retracted.

Preferably, the lancet drive train includes an adjustment screw which allows the user to set the extended position of the lancet. This enables the user to calibrate the operation of the meter such that his or her skin is punctured sufficiently to allow a large enough drop of fluid to form without causing too much pain.

Advantageously, the operation of the adjustment screw is arranged such that the distance of travel of the lancet remains constant, however much the extended position of the lancet is changed. This ensures that the amount of pain experienced by the user does not increase disproportionately to the depth of puncture.

Where the meter includes a lancet cap, it is preferable, as noted above, that the lancet cap provides a means for guiding the lancet drive train so that the lancet punctures ' the skin at approximately the same place on each actuation of the drive train.

Advantageously, in such case, the lancet drive train is arranged such that the end of the lancet, in its extended position, extends to approximately the plane formed by the pressure ring or aperture in the housing which, in use, is placed on the user's skin.

Preferably, the test strip cartridge comprises a cartridge housing defining a cavity configured to receive a stack of test strips, a partially detachable cartridge cap and a means for moving the stack of test strips towards the cartridge cap.

The test strips used for some measurements are air-or moisture-sensitive. It is therefore preferred that the cartridge includes a seal for sealing the cartridge cap to the cartridge housing when the cartridge cap is fully engaged with the cartridge housing. The seal may be on the cartridge cap or on the cartridge housing. Further, the cartridge may be manufactured from a material having dessicant properties, or a dessicant may be contained within the cartridge.

In use, upon activation of the meter, the cartridge cap is partially detached from the cartridge housing to allow the first test strip in the stack to be moved by the test strip dispensing system to the sample-receiving position. Once the measurement has been taken, the cartridge cap is preferably manually replaced on the cartridge housing to close the cartridge and seal its contents from atmospheric effects.

Preferably, the cartridge has on it data relating to the calibration code for the strips in the cartridge. The data may be present as visually readable indicia. In this case, the meter must include means, as mentioned above, to allow the user to enter the calibration code into the meter.

Preferably, however, the data on the cartridge are present in machine-readable format, for instance as a bar code or a resistance bridge circuit or stored in an electronic memory module.

If the data are present as a bar code, the meter will include a bar code reader. This may be a scanning reader or a stationary reader. A scanning reader will be more complicated but can be used when the cartridge is fitted in the meter. A stationary reader is less complicated but can only be used as the cartridge is inserted into or taken out of the meter.

If the data are present in an electronic memory module, this may comprise a read-only memory (ROM), or a rewritable memory, such as an EEPROM.

Preferably, the data also include a unique number identifying the specific cartridge, the number of strips originally present in the cartridge, the expiry date for the cartridge, different calibration factors for different sources of fluid (neonatal, arterial or venous blood, for instance) and any other relevant information such as control solution range information, preferably in machine-readable format, to assist in operation of the meter.

Where the memory module on the cartridge is rewritable, the meter may be arranged to write back into the memory module information such as the number of strips used, the date the cartridge was first used, the length of time the cartridge has remained open and the date, time and result of each test that was carried out with a strip from the cartridge.

Preferably, the test strip dispensing system includes a slider adapted to engage with only one of the test strips in the cartridge and move it to the sample-receiving position.

Advantageously, the meter includes a feeding channel which receives the strip from the cartridge and guides it to the sample-receiving position.

Preferably, the feeding channel includes a step arranged such, when the strip has been moved past the step, the strip drops, or is forced, into the step, thereby preventing the strip from moving back towards the cartridge.

Preferably, the strip is forced into the step by springs located on the meter. Advantageously, the springs are also electrically conductive and are arranged to make electrical contact with electrodes or a conductive bar on the strip (see below).

Preferably, the feeding channel is arranged such that the major plane of the strip, when in the sample-receiving position, is at an angle of from about 30° to about 60°, preferably about 45°, to the direction of movement of the lancet towards its extended position.

Advantageously, the meter includes an ejection means for ejecting a used test strip from the mete once a test has been completed. Preferably, where the cartridge includes a cartridge cap, the ejection means is operated as the cartridge cap is closed.

Preferably, the meter includes a deviator which prevents the test strip dispensing system moving a further test strip into the sample-receiving position while a first test strip is still in position. This is an advantageous feature as it allows the user to carry out a number of lancing operations with the same strip in position, since, in some cases, it takes a number of lancing operations, if necessary with adjustment of the lancet drive train, to produce a sufficiently large drop of fluid.

Preferably, the deviator operates in conjunction with the cartridge cap. While the cartridge cap is partially detached from the cartridge housing, the deviator blocks the normal path for the test strip dispensing system, such as the slider, and causes it to enter the cartridge cap.

Preferably, the meter includes a means for verifying that a strip is in the sample-receiving position. This may comprise a reflectance meter. Generally, test strips are more or less reflective than the surfaces of the feeding channel. Therefore, a change in reflectance will indicate that a test strip is in position.

Preferably, however, the verifying means comprises an electrical system. At its simplest, each strip may have on it a conductive bar arranged to short out two electrodes on the meter. This arrangement is useful for strips arranged to carry out photometric measurements.

Strips which are arranged to carry out electrochemical measurements already include electrode systems. Thus, the verifying means may include electrical contacts on the meter which contact the electrodes on the strip. Advantageously, as noted above, the electrical contacts on the meter are spring loaded and are positioned to force the strip into the step in the feeding channel.

Advantageously, the verifying means is also used to activate fully the circuitry in the meter. The meter may normally be in a low power mode, where the only active circuitry is that used to control the verifying means. Once the verifying means has indicated that a strip is present, the meter can then automatically switch to high power mode where all its relevant circuits are functioning.

Preferably, the verifying means is also arranged to start a timer in the circuitry of the meter. The timer is stopped by the ejection of a used strip from the meter, preferably by closure of the cartridge cap. This allows the circuitry to determine the length of time the cartridge has been open to the atmosphere. Advantageously the circuitry is arranged to sum the total time that the cartridge has been open and to produce a warning signal, such as an audible tone or a visible signal, if the total exceeds a pre-set maximum.

Preferably, the circuitry in the meter also counts the number of strips dispensed from each cartridge. Advantageously, the circuitry is designed to provide a warning signal, such as an audible tone or a visible signal, when the number of strips remaining the cartridge is low.

As noted above, the cartridge preferably includes a rewritable memory module and the circuitry in the meter is arranged to write back to the cartridge memory module useful information, such as the number of strips remaining in the cartridge and the length of time the cartridge has been open to the atmosphere. The rewriting function is particularly useful where a user is likely to be away from his normal environment for a length of time which would require the use of more strips than are present in a cartridge. In such cases, a user is likely to remove the old cartridge and insert a new, full cartridge. Once the new cartridge has been used up, the user may insert the old cartridge, even if it is out of date. As long as the meter can read the data on the old cartridge, it will be able to decide whether use of the old cartridge should be allowed.

Moreover, the provision of a rewritable memory module enables other possible uses. For instance, data on time and date of use and result of measurement may be written into the cartridge's memory module. The used cartridge could then be returned to the user's health care provider who could then study: the data to determine whether the user is complying with his treatment and monitoring regime. Alternatively, the used cartridges could be returned to the manufacturer to enable a general study of use to be carried out.

Preferably, the meter is activated manually by use of a single movement, for instance of a multi-functional handle assembly carried by the housing. The handle assembly may include a lever pivoted to the housing.

Preferably, the actuation of the handle assembly cocks the lancet drive train and moves a single test strip into the sample-receiving position. The movement of the handle assembly may also activate all the meter's circuitry.

The lancet drive train may be fired either by further movement of the lever or, preferably, by actuation of a trigger.

It can thus be seen that the use of the integrated sample testing meter of the present invention can be very simple. If desired, the user can replace an existing lancet with a new one. The meter can then be cocked by use of the handle assembly. This also moves a strip into the sample-receiving position. Movement of the lever or receipt of a strip in the sample receiving position also activates all the meter's circuitry. Then, the user only has to place the appropriate part of the meter, such as an aperture or the lancet cap, on his skin and activate the trigger.

If the first activation of the trigger does not cause the production of a sufficiently large drop of fluid, the meter can be cocked, positioned and fired again, if necessary a number of times, without the need to insert a new strip.

The presence of the pressure device ensures that, once a reasonable puncture is made, sufficient fluid will accumulate around the puncture so that it will contact the sample-receiving area of the strip and a sample will be taken onto or into the strip.

Thus, the use of the meter of the present invention avoids most of the steps presently required and in particular avoids those steps where manual dexterity and good eyesight are advantageous.

Preferably, the meter is adapted to produce and analyze a sample of blood or interstitial fluid, in particular to analyze a blood sample for glucose levels. Strips adapted to carry out such measurements are well known in the art. These may be electrochemical or photometric strips.

Advantageously, the strips are adapted to carry out electrochemical analyses and the circuitry in the meter is arranged to contact the electrodes in such strips.

Therefore, in a preferred embodiment the present invention provides an integrated blood glucose testing meter. The integrated meter of this aspect of the present invention allows for a simple, one-step glucose monitoring process, and significantly reduces the obstacles involved in frequent glucose monitoring. The integrated meter provides for the automated and precise dispensing and positioning of a test strip in repeatable close proximity to a lancet puncture site, automated transfer of a blood sample to the test strip and automated analysis of the blood sample after the test strip collects the sample from the puncture site.

In accordance with another aspect of the present invention, there may be provided a disposable test strip cartridge adapted to be loaded into the meter of the present invention. The cartridge includes a cartridge housing defining a cavity configured to receive a stack of test strips, a partially detachable cartridge cap and a stack of test strips disposed in the cavity.

According to another aspect, the present invention provides a multi-function handle assembly for the meter of the present invention. Preferably, the handle assembly comprises a lever. When a user depresses the handle assembly, the lever simultaneously cocks a lancing device and dispenses one test strip from the test strip cartridge into the sample-receiving position. The test strip is precisely positioned at a predetermined distance from the lancet.

According to yet another aspect, the present invention provides a strip dispensing system for forwarding test strips from a test strip cartridge to the sample-receiving position in the meter of the present invention on a one-by-one basis. The strip dispensing system includes a slider for pushing a test strip from the cartridge to a feeding channel and a deviator for preventing a plurality of test strips from being positioned in the feeding channel at one time. The deviator diverts the slider inside the test cartridge cap when a test strip is positioned in the feeding channel.

The present invention makes possible an integrated method of sampling and testing a blood glucose level or other analyte in a bodily fluid. Such an integrated method may comprise loading a test strip cartridge into an integrated testing meter, depressing a handle assembly on the testing meter to cock a lancing device and push a test strip into a sample-receiving position, pressing the integrated testing meter on the skin of a user and pressing a trigger of the testing meter to drive a lancet into the skin in order to form a drop of blood or other fluid on the skin surface. The test strip absorbs a required amount of blood or other fluid for an automated analysis of the sample by the integrated testing meter.

### Brief Description of the Drawings

These and other features and advantages of the present invention will be more fully understood by reference to the following detailed description in conjunction with the attached drawings in which like reference numerals refer to like elements through the different views.
Figure 1 is a perspective view of an integrated blood and testing meter according to the present invention.
Figure 2 is a plan view of the exterior of the integrated meter of Figure 1.
Figure 3 is a partially cut-away perspective of a disposable test strip cartridge that mounts in the meter of Figure 1.
Figure 4 is an exploded view of the components of the disposable test strip cartridge of Figure 3.
Figures 5a, 5b and 5c illustrate a "pop-up" feature of the cap of the disposable test strip cartridge.
Figure 6 is a partial cut-away view of one embodiment of the integrated meter in an idle position.
Figure 7 illustrates the integrated meter of Figure 6 when the multi-function handle is depressed.
Figure 8 illustrates the integrated meter of Figure 6 when the multi-function handle is released.
Figure 9 illustrates the integrated meter of Figure 6 when the multi-function handle is pressed when a test strip is loaded.
Figure 10 is a side view of a lancet cap suitable for use with the meter of the present invention.
Figure 11 is an end view of the lancet cap of Figure 10.
Figure 12 is a side elevational view in cross section along line 4-4 of Figure 11, illustrating the contact ring of the cap of Figure 10.
Figure 13 is a graphic representation of the pressure profile created by the cap of Figure 10.
Figure 14 is a cross-sectional view of an alternate embodiment of the contact ring of the lancet cap for use with the meter of the present invention.
Figure 15 is a side elevational view of an alternative embodiment of a lancet cap for use with the meter of the present invention, illustrating a sleeve positioned about the cap.
Figure 16 is an end view of the lancet cap of Figure 15.
Figure 17 is a side elevational view of the lancet cap of Figure 15, illustrating the cap displaced from the skin.
Figure 18 is a side elevational view of the lancet cap of Figure 15, illustrating the cap in contact with the skin.
Figure 19 is a front elevational view of the cap of Figure 15.
Figure 20 is a front elevational view of another embodiment of a lancet cap for use with the meter of the present invention.
Figure 21 is an end view of the cap of Figure 20.
Figure 22 is a front elevational view of another embodiment of a lancet cap for use with the meter of the present invention for lancing the ventral side of a fingertip.
Figure 23 is an end view of the lancet cap of Figure 22.
Figure 24 is a side view of an alternate embodiment of a lancet cap for use with the meter of the present invention formed of a flexible material and disposed in a rest position.
Figure 25 is a side view of the lancet cap of Figure 24 when the contact ring of the lancet cap contacts a lancing site.
Figure 26 is a side view of an alternate embodiment of a lancet cap for use with the meter of the present invention formed of a deformable, flexible material and disposed in a rest position.
Figure 27 is a side view of the lancet cap of Figure 26 when the contact ring of the lancet cap contacts the lancing site.
Figure 28 illustrates the feeding channel of the integrated meter according to the present invention.
Figure 29 is a schematic view of a test strip design suitable for use in the present invention.
Figure 30 is a schematic representation of the electronics which can be incorporated in an integrated meter in accordance with the present invention.
Figure 31 illustrates an alternative mechanism for providing a "pop-up" feature of the cap of the disposable test strip cartridge, with the cap closed.
Figure 32 illustrates the mechanism of Figure 31 with the cap open.
Figure 33 illustrates the mechanism of Figure 31 from an opposite side.
Figure 34 is an opposite side view of the mechanism of Figure 31 with the cap open.

### Detailed Description of the Invention

The present invention provides an integrated meter for sampling and analyzing a sample of bodily fluid, such as blood, including a disposable test strip cartridge having a stack of test strips disposed therein. The present invention facilitates the monitoring of, for instance; blood glucose levels by integrating into a single meter the steps involved in sampling and analyzing blood into a simple process employing a single meter.

The present invention will be described below relative to an illustrative embodiment. Those skilled in the art will appreciate that the present invention may be implemented in a number of different applications and embodiments and is not specifically limited in its application to the particular embodiment depicted herein.

The present invention will be discussed below in connection with sampling blood, although those of ordinary skill will recognize that other types of fluid can also be used.

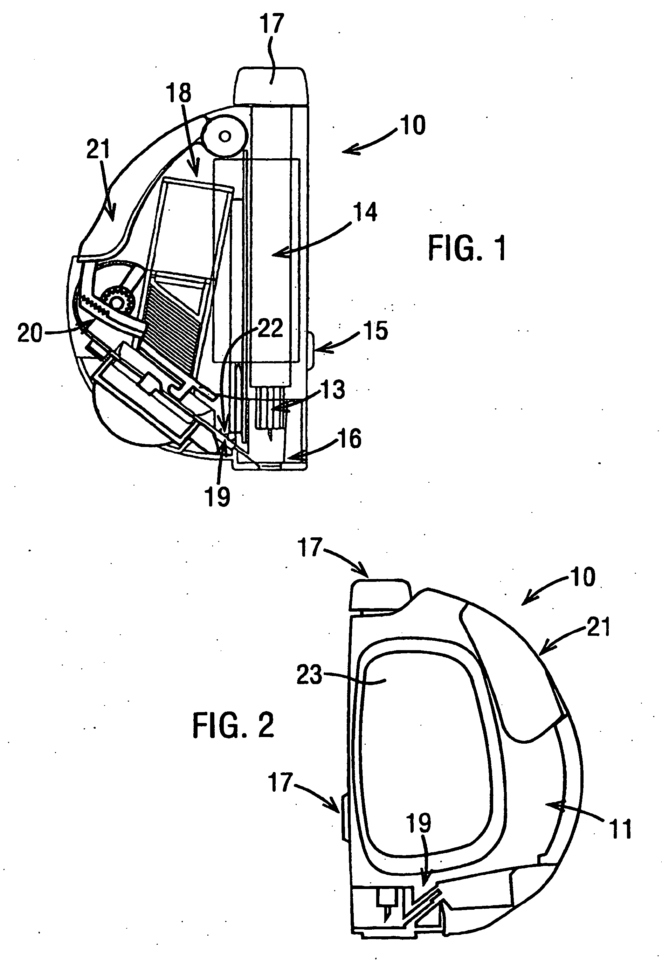

Figures 1 and 2 illustrate an integrated blood glucose sampling and testing meter 10 according to an illustrative embodiment of the present invention. This meter is designed to carry out electrochemical analysis of a blood sample. However, if desired, the same mechanical parts could be used in connection with photometric analyses. The sampling and testing meter comprises a modular housing 11 (shown in Figure 2) encompassing an integrated system for expressing and subsequently analyzing a sample. The meter 10 includes a lancet assembly for puncturing the skin of a user to express a drop of blood on the surface of the skin. The lancet assembly includes a lancet 13 and a lancet drive train 14 for driving the lancet into and out of the skin. When cocked, a triggering button 15 actuates the drive train.

A lancet cap 16 is removably attached to the housing 11 at the proximal end of the device 10 and includes an aperture to allow passage of the lancet 13 through the cap 16 and into the skin of the user. The cap 16 can have a multi-contoured surface in order to promote, enhance or facilitate the expression of blood by pressing the device onto the skin. The lancet assembly further includes a depth adjustment knob 17 situated at the distal end of the drive train opposite the lancet. Rotation of the depth adjustment knob decreases or increases the puncture depth of the lancet. The depth adjustment knob regulates or adjusts the puncture depth in accordance with known techniques.

A test strip cartridge 18 is loaded into the meter 10 and includes a stacked supply of test strips disposed within a cavity or hollow interior of the cartridge housing. The test strip cartridge is adapted to dispense individual test strips to a feeding channel 19. The outlet of the feeding channel leads into the interior of the lancing cap 16, in precise and close proximity to the aperture through which the lancet 13 passes when puncturing the skin. In this manner, when the lancet punctures the skin and a drop of blood forms on the skin surface, the test strip is located in close proximity to the puncture wound to ensure that blood contacts the strip. Moreover, the precise positioning of the test strip adjacent the lancet ensures that only small volumes of blood are required, as the strip is able to touch and automatically wick up even a small drop of blood.

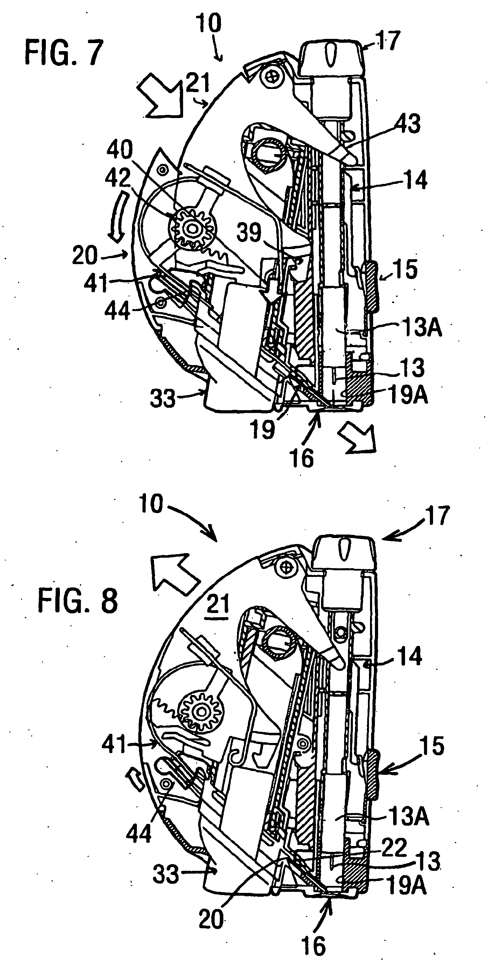

According to one practice, as illustrated in Figures 7, 8 and 9, the housing includes an internal wall 19A that defines the inner side of the lancet cap 16. According to an alternate embodiment, the housing wall 19A can have a frusto-conical or funnel shape, or any other suitable shape, for precisely positioning the lancet relative to the test strip.

The lancet cap is precisely dimensioned such that the lancet body 13A that carries the lancet 13 slidably passes through the cap. In this arrangement, the lancet 13 is precisely positioned at about the same location each time it is deployed. Correspondingly, each test strip is precisely positioned at about the same location each time one is moved from the cartridge to the sample-receiving position.

The terms "precise" and "precisely" are used herein to include positioning the puncture point of the lancet 13 and the sample-receiving area of the test strip relatively close to each other in a repeatable manner.

The integrated meter of the present invention is able fully to exploit the technological improvements in strip design which allow the use of much smaller samples. Presently available strips require only 1 to 3 µl of sample. Many users have eyesight problems and cannot see well enough to be able to transfer such small volumes of sample accurately to the sample-receiving area of a strip. Such eyesight problems are common complications of diabetes. The automatic collection and transfer of samples to the strip enabled by the present meter is a major advantage of the present meter.

It is thus possible to arrange the operation of the lancet and the pressure device to produce a puncture which allows a small, but sufficient, volume of blood to be expressed as a drop of the user's skin. Such a small drop will be able to contact the sample-receiving area of the test strip due to the precise positioning of the lancet and the test strip.

The small volume of blood or other bodily fluid expressed from the user is sufficient to accurately determine or monitor the presence or absence of an analyte, such as glucose.

The meter is arranged such that, in use, with the strip in the sample-receiving position, its sample-receiving area is separated from the puncture by between about 0.4 mm and about 1.3 mm, and preferably between about 0.7 mm and about 0.9 mm.

Once the sample drop touches the sample-receiving area of the test strip, the test strip automatically directs the blood sample to the testing portion of the strip.

The test strip cartridge comprises a replaceable and disposable portion of the sampling and testing meter. When the supply of test strips is depleted or expired, the user may remove the used test strip cartridge 18 and insert a new test strip cartridge containing a fresh supply of test strips. The details of the test strip cartridge 18 are described in depth below.

A strip dispensing system 20 operates in co-operation with the test strip cartridge 18 to dispense test strips one-by-one through the feeding channel 19 and into the sample-receiving position to effect the sampling and analysis of a blood sample. According to the illustrated embodiment, when a user depresses or squeezes the handle 21 of the device 10, the strip dispensing system 20 pushes the foremost test strip in the stack out of the test strip cartridge and into the feeding channel. According to a preferred embodiment, handle 21 performs an additional function of simultaneously cocking the lancet assembly to prepare the lancet assembly for lancing the skin of a user when the user depresses or squeezes the handle. The workings of the strip dispensing system and multi-function handle 21 are described in further detail below.

To enable electrochemical analysis of the sample, the meter further includes electrical contacts 22 situated adjacent the feeding channel 19 and configured to contact electrodes formed on the test strip. The electrical contacts connect to electronics (not shown) located within the modular housing 11 of the sampling and testing meter. The electronics are arranged such that, once the contacts 22 contact the electrodes in the strip, the meter switches from "low" power made to "high" power mode.

The test strip generates electrochemical signals that are passed by the electrical contacts to the housing electronics. The electronics process the signal and calculate the glucose level or other electrochemically detectable analyte of the blood or other interstitial fluid that is sampled by the testing device. The electronics transmit instructions for an appropriate display or output regarding the analysis.

The feeding channel 19 has in it a step 60 located adjacent the electrical contacts 22. The electrical contacts 22 are spring biased so that, once a test strip is in the sample-receiving position, the electrical contacts 22 bear on the test strip and locate it securely in the step. In this manner, the strip is prevented from moving backward away from the sample-receiving position.

As illustrated in Figure 2, the integrated sampling and testing meter 10 includes a visual LCD display 23 for displaying information related to the analysis of the sample. According to the illustrative embodiment, the information in the display includes a measured blood glucose level in a blood sample, as well as the time and date of the measurement. The display may also provide information regarding the number of test strips remaining in the test strip cartridge, the operating temperature, the expiration date of the test strip cartridge, instructions to the user and the like. According to one practice of the invention, test results are stored in memory in the meter and the display 23 allows a user to view prior test results.

The meter also has on its outside buttons which can be used by the user to enter data into the meter's electronics. This may be achieved by using the buttons to navigate through one or more menus displayed on the display 23.

To measure blood glucose levels with the integrated meter 10, a user first depresses the handle 21 to simultaneously cock the lancet assembly and automatically open the test strip cartridge and to advance a test strip from the cassette to the feeding channel 19. The user then presses the meter 10 against a body part, such as a finger or forearm, such that the skin of the user contacts the lancing cap 16. When a user pushes the trigger button 15, the lancet assembly fires the lancet 13 into the skin a predetermined depth and at a precise location. The lancet assembly immediately retracts the lancet from the skin.

The lancing cap 16 includes a pressure ring (not shown) so that, as the meter is pressed onto the skin after the lancing has taken place, a drop of blood of the required size forms on the user's skin. As the blood drop grows, the blood contacts the sample-receiving area of the test strip and capillary force absorbs blood into the strip for analysis. The user holds the meter firmly against the skin until a sufficient amount of blood is absorbed into the test strip, generally for about 3 to 10 seconds. According to one practice, the meter 10 produces an audible or visible signal to the user indicating that a sufficient blood sample has been collected and that analysis has begun. The user then removes the meter from the skin and the electrochemical analysis of the sample continues until the result is displayed.

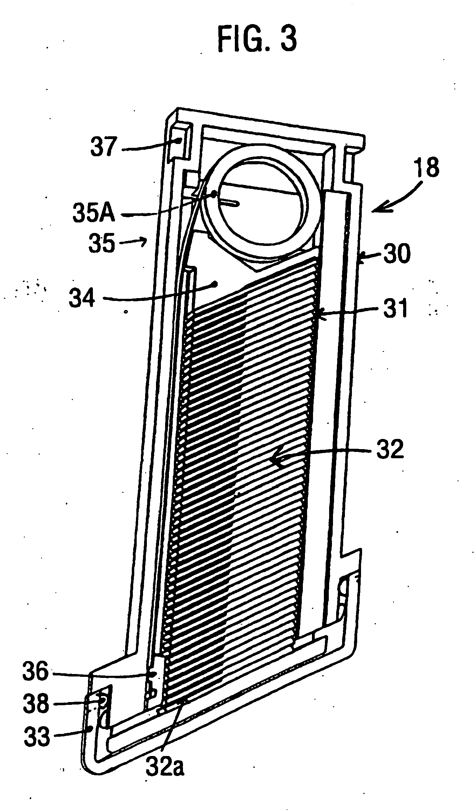

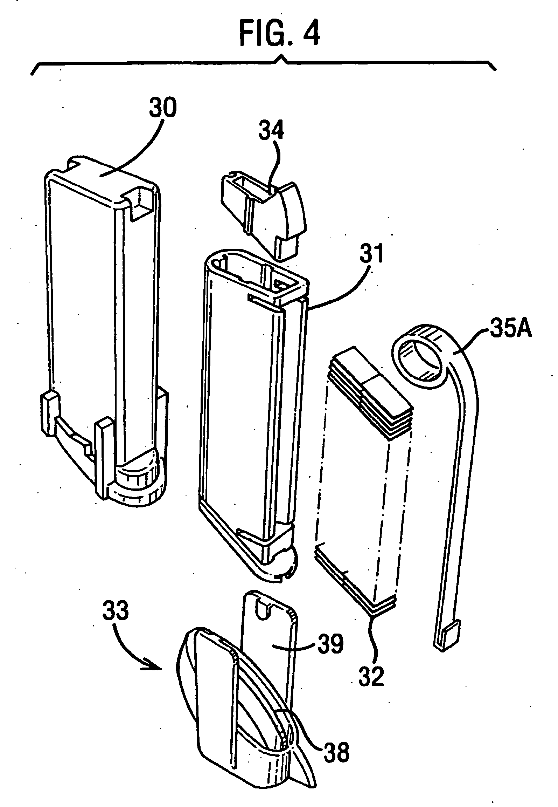

Referring to Figures 3 and 4, the disposable strip cartridge 18 includes a number of components designed to facilitate automatic, one-by-one dispensing of the test strips. According to the illustrated embodiment, the test strip cartridge includes a vial housing 30, a cartridge housing 31 including a stack of test strips 32, a cartridge cap 33 and a push-up or biasing mechanism 35. It is readily seen that the cassette housing 31 is disposed within the vial housing 30 in use, and that the cartridge cap 33 acts to close the vial housing. The push-up mechanism 35 is also disposed within the vial housing. As shown, the stack of test strips comprises about fifty test strips in vertical alignment. However, the test strip cartridge of the present invention is not limited to a stack of fifty test strips and may include any number of stacked test strips.

The push-up 35 mechanism biases the test strip stack 32 towards the cartridge cap 33 such that when a foremost test strip 32a is removed from the stack, the remaining test strips in the stack advance by one. After the foremost strip is removed from the stack, the next strip in the stack moves up and is ready to be dispensed for a subsequent analysis.
The push-up mechanism includes a loader 34 pressing against the last strip in the stack 32 and a biasing element, illustrated as tensator 35A. As illustrated, the tensator 35A comprises a constant force clock spring that applies a constant pressure to the stack. The push-up mechanism may also be provided with a non-return ratchet mechanism, or the like, active upon the loader 34 to prevent the loader 34 from moving back down within the cassette housing 31 and thus causing the top strip 32 to leave the ready-to-be-dispensed position. The ratchet may take the form of an array of appropriately shaped protuberances disposed inside the cassette housing 31 and co-operative with protuberances upon the loader 34 exterior.

The push-up mechanism 35 further includes a tensator retainer 36 to secure a portion of the tensator to the cassette housing. The vial housing 30 further includes notches 37 to releasably lock the cartridge in the modular housing of the meter. When loading the cartridge 18 into the meter the vial housing clicks unambiguously in place to ensure a precise fit.

The cartridge cap 33 includes a hermetic sealing element 38 which contacts the vial housing to form a seal to protect the test strips from humidity, which can damage the test strips and compromise test results. Alternatively, the seal can be included in the vial housing where it meets the cap.

According to one practice, the cartridge material itself can have desiccant properties, or desiccants can be disposed in the interior space of the vial. Any humidity that may migrate into the test strip vial is by these materials absorbed and neutralized.

Preferably, the cartridge includes on it a re-writable memory module such as an EEPROM chip. In this case, the electronics in the meter will include means for interfacing with the memory module so that the meter can read from and write to the memory module.

The memory module will contain a calibration code for the cartridge and will preferably contain a unique code for the cartridge and its expiry date. It may also contain compensation factors for analyses of different fluids (such as venous, arterial or neonatal blood or interstitial fluid), the number of strips in the cartridge and other relevant information. The electronics in the meter will be set up to use any data stored in the memory module, in particular the calibration code.

The electronics will also be set up to write to the memory module such information as the number of strips used, the length of time the cartridge cap has remained open, the date of first opening the cap, the date and times of each test carried out and the result of the test.

The cartridge may alternatively include such data in other formats, such as in visible characters, as a bar code or as a resistor bridge circuit.

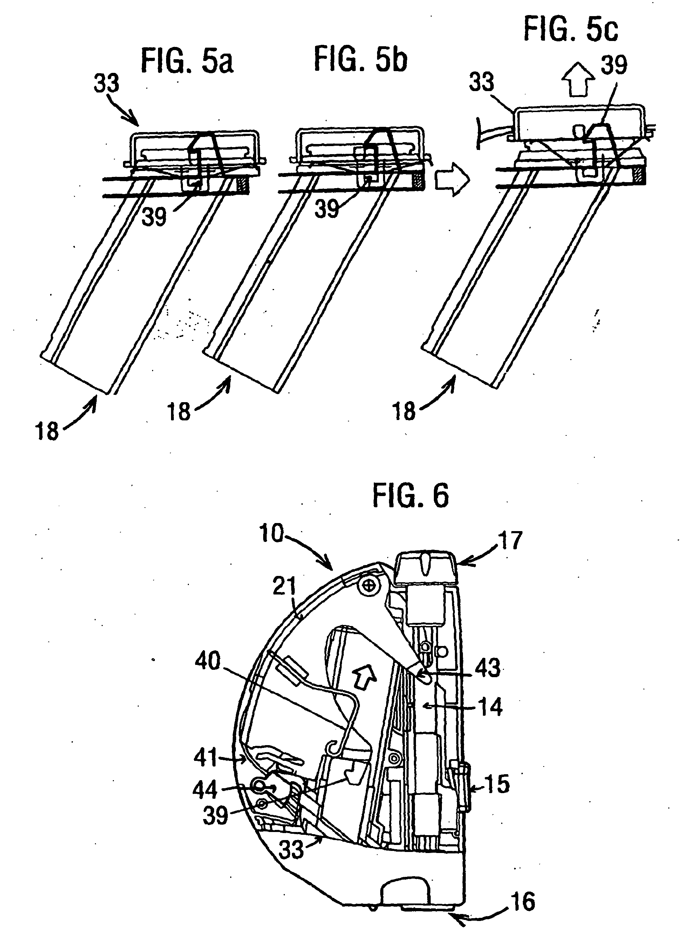

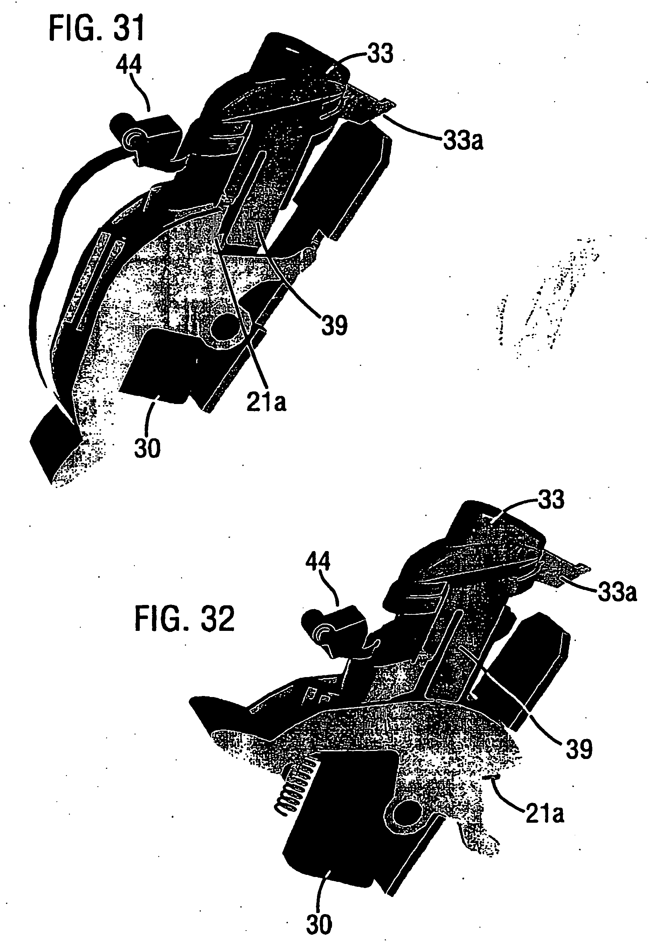

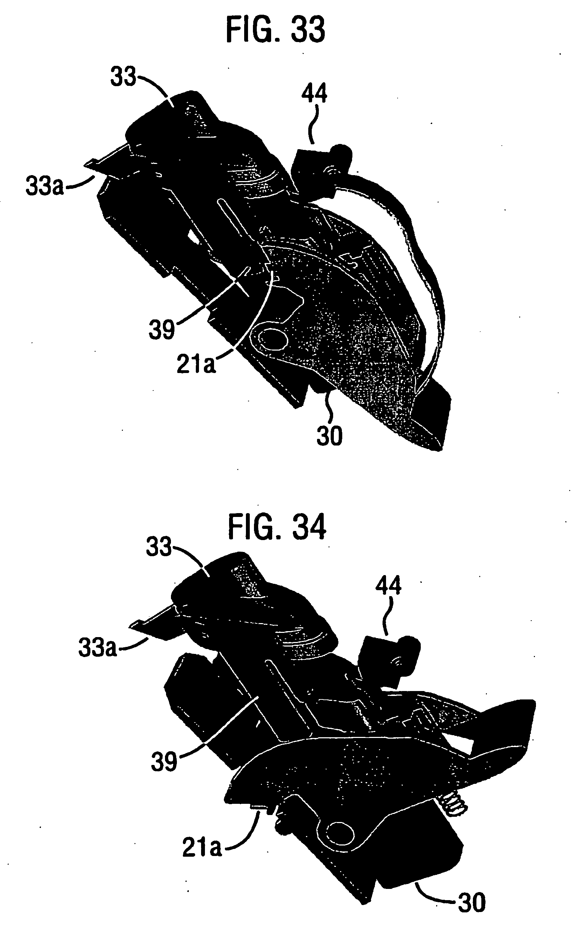

As shown in Figures 4, 5a, 5b, 5c, 31, 32, 33 and 34 the cartridge cap is releasably locked into place on the cartridge by a cap retainer 39. To allow for the strip dispensing mechanism in the meter to forward individual strips to the feeding channel 19, the cartridge cap 33 includes a "pop-up" feature. The cartridge cap 33 is flexibly attached to the vial housing 30 by means of side supports, hinges, springs or another suitable mechanism. Pushing the cap retainer 39 releases the lock on the cartridge cap and allows the cap to pop up a predetermined amount, thereby allowing the foremost test strip 32a in the stack 32 to be fed to the sample-receiving position. As may be seen in Figures 5a to 5c, the lock may be released by pushing the locking member 39 such that it moves as a whole, thereby moving out of locking contact with the cap 33. Alternatively, as seen in Figures 31 to 34 the retainer or retainers 39 may be bent away from the vial housing 30, by an outward pressure exerted by one or more arms 21a inserted between the retainer 39 and the vial housing 30 for example, thereby releasing their locking hold and allowing the cap 33 to "pop-up".

The strip dispensing system 20 of Figure 1 cooperates with the pop-up cartridge cap described above to push the foremost test strip of a test strip stack into the feeding channel 19 in order to position the test strip in the sample-receiving position with its sample-receiving area in close and precise proximity to a puncture site. As discussed, the strip dispensing system 20 comprises a multi-function handle 21.

Figure 6 is a view of the integrated meter in an idle position. Figure 7 illustrates the integrated meter when the multi-function handle is depressed. In the idle position, the cap retainer 39 forces the cartridge cap 33 closed and securely connects the cartridge cap 33 to the body of the test strip cartridge. The multi-function handle 21 comprises a lever 21a (see also Figures 31 to 34) and when a user depresses the lever, the lever disengages the locking mechanism on the cartridge cap 33. As illustrated, a cap opening spring 40 inside the handle lever 21 pushes on the cap retainer 39 to release the cap 33 from the cartridge body and expose the slider slot behind the foremost test strip within the test strip cartridge. At the same time the handle lever 21a actuates a slider 41, which slides behind the foremost test strip and pushes the foremost test strip into the feeding channel 19 of the meter 10.

According to the illustrated embodiment, the slider is a rotating flexible slider 41 and the drive element for the slider 41 includes a device, illustrated as a ratchetwheel in cooperation with a cog 42, to ensure that the foremost test strip is completely pushed into the feeding channel before another test strip can be dispensed. The slider 41 only returns to its original position when the foremost test strip has been properly and accurately placed into the feeding channel and is in the sample-receiving position.

Those skilled in the art will recognize that any suitable mechanism may be utilized for forwarding a test strip into a feeding channel and ensuring that the test strip is entirely dispensed. Once in the feeding channel, the test strip is positioned to receive a blood sample for analysis. After the analysis is complete, the user replaces the cartridge cap 33 to re-seal the cartridge. According to one practice of the invention, closing the cartridge cap 33 ejects the used test strip from the meter 10. This may be achieved by a contoured protrusion 33a from the cap 33, as shown in Figures 31 to 34. The protrusion may be in abutment with the rear of the in use strip or may be brought into abutment therewith during closing of the cartridge cap 33. In this embodiment, the protrusion is contoured to progressively force the strip out of its sample-receiving position as the cap 33 closes, thereby ejecting the used strip and clearing the way for a new strip to be loaded during the next operation of the apparatus.

As illustrated, the multi-function handle 21 further includes a cocking lever 43 for arming the drive train 14 of the meter 10 when the handle is depressed. According to one practice, the multi-function handle further operates to switch on the electronics (not shown) of the meter 10 to prepare the meter for analysis of a prospective blood sample. According to an alternate embodiment, the electronics include a strip detector for detecting the presence of a test strip in the feeding channel. Thus, when the strip detector detects a strip in proximity to the lancing site, the electronics switch on.

According to one aspect, the strip dispensing system 20 is designed to ensure that only one test strip is loaded at a time. The strip dispensing system 20 includes a deviator 44 in co-operation with the slider 41. The strip dispensing system 20 allows only one test strip to be forwarded at a time. After the handle 21 is released and the slider 41 is brought back into its initial position, as shown in Figure 8, the deviator 44 automatically rotates into a position to deflect subsequent attempts to load an additional test strip into the feeding channel. The release of the cartridge cap caused by depression of the handle allows the deviator to rotate once the slider is moved back to its idle position. After forwarding one test strip, the slider route is deviated inside the cartridge cap 33, rather than through the test strip cartridge and into the feeding channel of the meter 10.

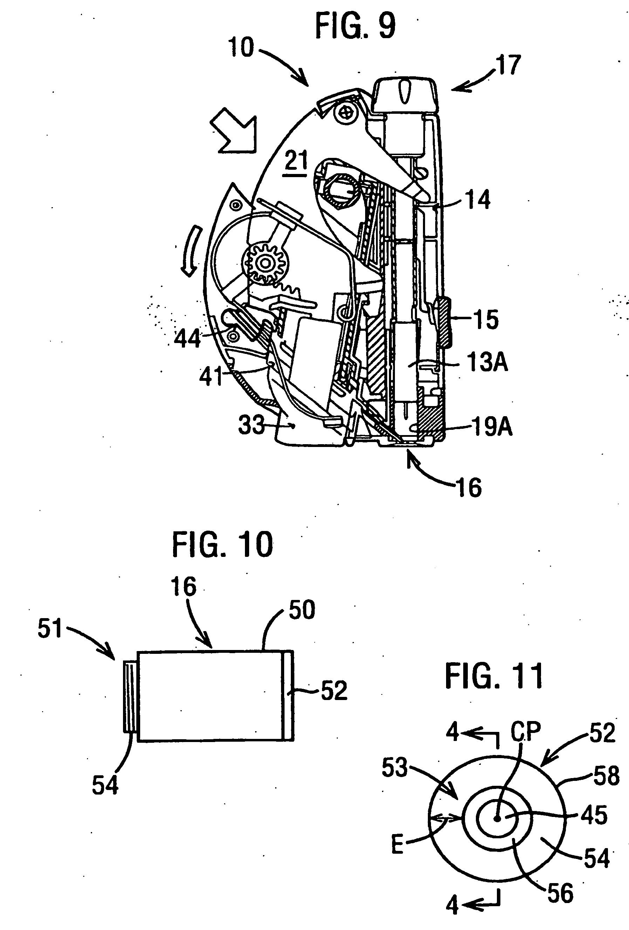

When the user presses the cartridge cap 33 closed, thereby ejecting the used test strip, the deviator rotates back and resets the strip dispensing system to dispense a new strip. As illustrated in Figure 9, if a test strip is already loaded into the feeding channel 19, additional squeezing of the handle 21 only serves to cock the lancet assembly 14 and does not load another test strip into the channel. In this manner, the strip dispensing system 20 allows several cocking and lancing attempts using the same test strip. This feature is particularly useful if the lancet is accidentally discharged or if the lacing action does not generate a sufficient amount of blood. In this case, the lancet assembly can be re-cocked without wasting a test strip.

The test strip cartridge 18 and the strip dispensing system 10 co-operate with the lancet assembly illustrated in Figure 1 to efficiently and less painfully obtain and analyze a blood sample from a user. As discussed above, squeezing handle 21 simultaneously cocks the lancet assembly and forwards a test strip from the cassette into the feeding channel 19. Referring again to Figure 6, the lancet assembly is in a neutral position prior to sampling. The drive train 14 of the lancet assembly comprises a drive tube, a lancet holder slidably mounted in the drive tube for holding the lancet 13, a first spring for urging the lancet holder forward, a second spring for retracting the lancet 13 after the lancet punctures the skin, a trigger button 15 and a depth adjuster knob 17. The lancet assembly further includes a lancet cap 16 having an aperture for guiding the lancet 13 through the aperture to the skin of a user and for shielding the lancet when not in use. Initially, the foremost test strip 32a within the test cartridge is also in a neutral position.

In one embodiment, the first spring is a hard spring and the second is a soft spring. In this instance, the residual momentum of the lancet, supplied by the first spring upon urging forward the lancet, causes the compression of the second spring. This compression begins when penetration of the skin by the lancet occurs. Once the force of the second spring on the lancet outweighs the residual momentum, the second spring causes the retraction of the lancet.

When the handle lever 21 is depressed, as illustrated in Figure 7, the cocking lever 43 retracts the drive tube to arm the lancet assembly, while simultaneously the test strip is fed through the feeding channel 19 and into the sample-receiving position within the lancet cap 16. The test strip is precisely located relative to the lancet 13. The user presses the lancet cap 16 against a body part, such as a finger or arm, and depresses the trigger button 15 to allow the lancet assembly to drive the lancet tip into the skin, in close proximity to the sample-receiving area of the test strip. The lancet assembly subsequently retracts the lancet tip from the skin.

The pressure ring squeezes the skin to maximize the quantity of blood formed from a puncture. Once the drop of blood is large enough, it will touch the sample-receiving area of the strip and will be wicked into the strip. The test strip automatically directs the blood sample to an analysis portion and the analysis of the blood sample begins automatically.

After the analysis is complete, the user may remove the lancet cap 16 and the lancet 13 from the lancet holder. The user may then discard the lancet 13, if desired. Those skilled in the art will recognize that alternate lancet assemblies may be utilized in accordance with the teachings of the present invention. For example, the present invention is not limited to the dual-spring drive train of the illustrative embodiment of the invention.

Figures 10 through 27 illustrate different embodiments of the design for the lancet cap 16 of the present invention. The cap 1.6 for the integrated meter 10 includes a cap body 50 having a proximal end 51 for connecting to the housing 11 of the lancing device and a contact ring 52 attached to the distal end of the cap body. The contact ring 52 includes an aperture 45 for a portion of the lancet 13 of the lancing device to pass therethrough. The contact ring 52 has a multi-contoured surface 53 oriented generally about an axis distinct from the axis of motion of the lancet 13. The multi-contoured surface is designed to pressure the dermal tissue to facilitate expression of a fluid sample after lancing the dermal tissue.

Referring to Fig. 10, the cap body 50 can include a connector 54 for removably and replaceably connecting the proximal end of the cap body to the housing 11 of the integrated meter 10. The connector 54 preferably is threaded to mate with corresponding threads provided in the housing 11 of the device 10. One skilled in the art will recognize that alternative connecting mechanisms may be used without departing from the scope of the present invention. For example, the connector 54 can be sized and shaped to snap-fit to the housing 12. In addition, the cap 16 can be permanently affixed to the housing 11, although it is preferable for the cap to be removably and replaceably connected to the housing 11. The cap body 50 and the contact ring 52 can be constructed from plastic or other materials suitable for use in a medical instrument.

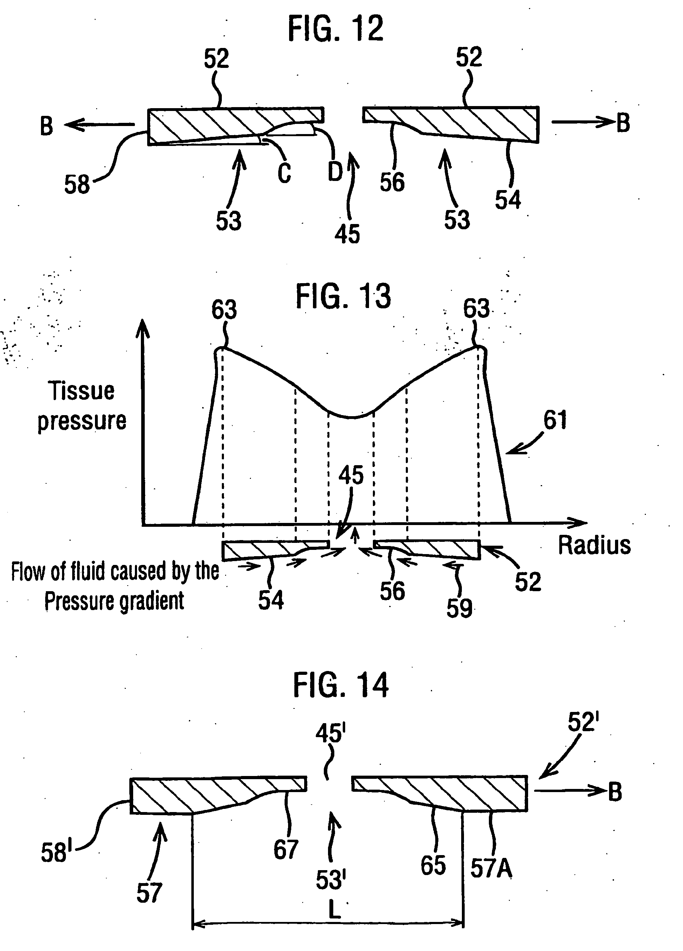

Referring to Figures 11 and 12, the contact ring 52 preferably has a multi-contoured surface 53 for contacting the dermal tissue both during lancing and during blood sample expression. The multi-contoured surface 53 is oriented generally about an axis, indicated by line B in Figure 12. According to the illustrative embodiment, the lancet travels along a first axis and the multi-contoured surface is oriented about a second axis perpendicular to the axis of motion of the lancet. One skilled in the art will recognize that the second axis is not limited to this preferred orientation and that any orientation distinct from the axis of motion of the lancet can be employed.

The multi-contoured surface 53 is designed to pressure the dermal tissue to maximize blood flow rate from the periphery of the pressured area to the center of the lancing site and to facilitate the expression of a blood sample for collection. The term multi-contoured surface as used herein can comprise two or more surfaces oriented at distinct angles with respect to each other and with respect to a common axis. The multi-contoured surface can extend inwardly from a vertical wall, or can extend inwardly from a flat surface extending radially inwardly from the vertical wall. Those of ordinary skill will recognize that the multi-contoured surface can include any selected number of surfaces. The surface can be, according to one practice, non-planar. In one embodiment described herein, the multi-contoured surface 53 is comprised of an outer radial portion 54 and an inner radial portion 56 proximate the opening 45. The outer radial portion 54 is preferably oriented at a first angle C relative to the second axis B. The inner radial portion 56 is preferably oriented at a second angle D, distinct from the first angle C, relative to the axis B. The outer radial portion 54 and the inner radial portion 56 can have any selected surface feature or shape, e.g. can be linear, stepped or curved. In the embodiment illustrated in Figure 12, the outer radial portion 54 is generally linear from the perimeter 58 of the contact ring 52 to the intersection with the inner radial portion 56. Alternatively, the outer radial portion 54 can be convex or concave in curvature. Additionally, the inner radial portion 56 is generally concave in curvature, but can also be linear or convex.

In an illustrated embodiment, the angle C, corresponding to the slope of the outer radial portion 54, is in the range between about 5° and about 15°. Additionally, the radial extent of the outer radial portion 54, generally illustrated by line E in Figure 11, is preferably about 25% to about 75% of the total radius of the contact ring 52, as measured from the center point CP of the contact ring 52 to the perimeter 58 of the contact ring 52. In a preferred embodiment, the radial extent E of the outer radial portion 54 is preferably about 50% of the total radius of the contact ring 52.

The contact ring 52 can be a separate, discrete component affixed to the cap body 50 or can be integrally formed with the cap body 50.

With reference to Figure 13, the contact ring 52 of the cap 16 is sized and dimensioned to be placed in intimate facing contact with the skin of the user. When placed thereagainst, the contact ring creates a pressure gradient that extends from the radial outer surface inwardly towards the opening 45. Specifically, when the skin is lanced by the lancet 13, the contact ring 52, which is disposed about the lancing site, creates a pressure gradient that urges fluid to flow toward the opening 45, as indicated by arrows 59.

The pressure profile 61 created by the cap 16 has pressure peaks 63 that coincide with the perimeter portion of the cap, or with the start of the multi-contoured surface 53. The pressure is a maximum at this portion since the cap contacts the skin of the user to a greater degree. When the surfaces of the multi-contoured surface extend inwardly towards the opening 45 and away from the skin, the overall pressure decreases. This forms a pressure gradient that extends from the outermost portion of the cap 16 to the opening 45. Those of ordinary skill will recognize that the pressure profile will change as a function of the configuration of the contact ring.

Fig. 14 illustrates another embodiment of the contact ring 52 of the cap 16 of the present invention. Like reference numerals designate like or similar parts plus a superscript prime. The illustrated contact ring 52' has an axially or vertically extending outer wall or perimeter 58' that terminates at a distal end 57. The distal end 57 includes a first flat face portion 57A that is adapted to press against the skin of the user during use. The flat face portion 57A is generally perpendicular to the perimeter portion 58'. The multi-contoured surface 53' extends radially inwardly from the flat face portion 57A towards the opening 45'. The multi-contoured surface 53' extends between the annular flat face portion 57A, as indicated by the designation L.

The illustrated multi-contoured surface 53' includes two or more surfaces oriented relative to each other to form different, distinct angles. In particular, the multi-contoured surface 53' includes a pair of surfaces 65 and 67. The radially outer surface 65 is oriented at a first angle relative to the axis B. The radially inner surface 67 is oriented at a second angle relative to the axis B different from the first angle. As described above, the surfaces 65 and 67 can have any selected shape or angle.

In use, the cap 16 is connected to the housing 11 of the meter 10 and the dermal tissue is lanced by the lancet 13 passing through the opening 45 in the contact ring 52. The lancet 13 is then withdrawn into the housing 11. The contact ring 52 is pressed into contact with the dermal tissue proximate the lancing site causing blood to exit the lancing site and enter the cap 16 through the opening 45. Dermal tissue is "squeezed" into contact with the outer radial portion 54 and the inner radial portion 56 of the multi-contoured surface 53. The multi-contoured surface 53 facilitates blood expression by increasing the pressure on the dermal tissue in contact with the perimeter 58 of the contact ring 52. The pressure on the dermal tissue decreases as the slope of the outer radial surface 54 and the inner radial surface changes toward the opening 45. This inwardly extending pressure gradient is illustrated in Figure 13.

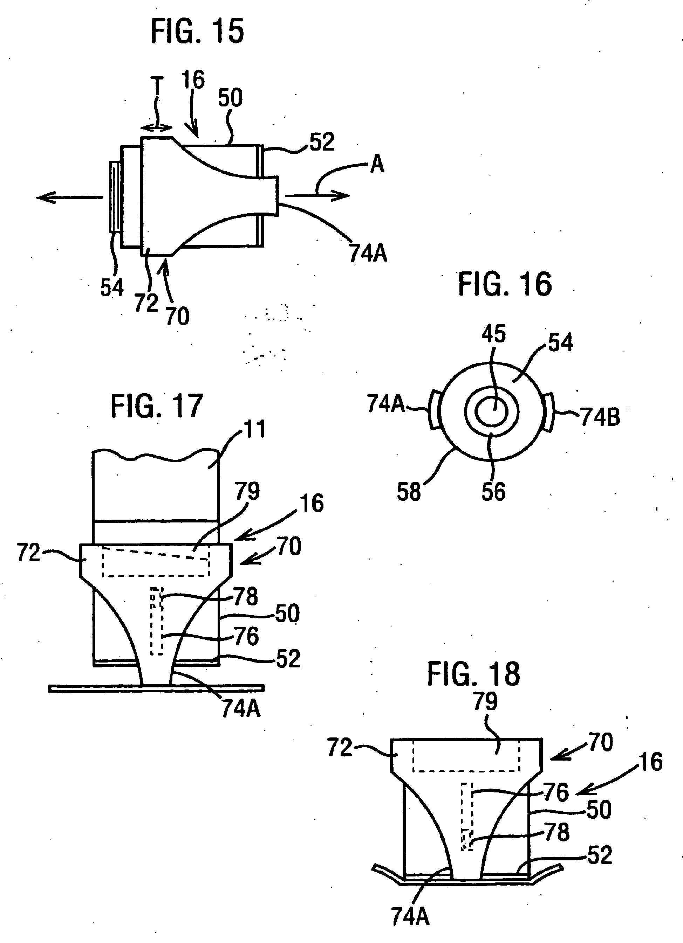

An alternative embodiment of the cap is illustrated in Figures 15 through 19 in which a sleeve 70 is mounted about the cap body 50. The sleeve 70 is movable generally along a first axis A, i.e. along the axis of motion of the lancet, and relative to the cap body 50. The sleeve 70 comprises an annular collar 72 and at least two legs 74A and 74B that extend from the collar 72 in the direction of the first axis A toward the distal end of the cap 16. The legs 74A and 74B taper from an increased width proximate the collar 72 to a decreased width proximate the contact ring 52.

As illustrated in Figure 16, the legs 74A and 74B are arcuate in cross-section and encompass only a portion of the circumference of the contact ring 52. The legs 74A and 74B are preferably symmetrically disposed about the circumference of the contact ring 52. Although only two legs are illustrated in the Figures, one skilled in the art will appreciate that additional legs can be added without departing from the present invention. In addition, the legs need not be positioned symmetrically about the contact ring 52.

The sleeve 70 is preferably slidable along an axis parallel to the first axis A, as indicated by arrow T in Figure 15. A longitudinally extending slot 76 can be formed in one or both of sides of the cap body 50. A protruding guide member 78 can be formed in one or both of the legs 74A and 74B. The guide member 78 is sized and shaped to slide within the slot 76 and inhibits lateral motion of the sleeve 70 relative to the cap body 50. Alternatively, the slot 76 can be formed in one or more of the legs 74A and 74B and the guide member 78 can be formed on the cap body 50.

A spring 79 or other biasing mechanism can be provided to bias the sleeve 70 toward the distal end of the cap 10.

It is sometimes desirable to remove the cap 16 and the contact ring 52 from contact with the dermal tissue after lancing, for example, to remove pressure from the dermal tissue or to visibly inspect the lancing site. The sleeve 70 allows the user to maintain a portion of the lancing device, the legs 74A and 74B of the sleeve 70, in contact with skin when the cap 16 and the contact ring 52 are removed from contact with skin, as illustrated in Figure 17. Importantly, the legs 74A and 74B allow the user to maintain the opening 45 in alignment with the lancing site when the contact ring is returned into contact with dermal tissue, as illustrated in Figure 18.

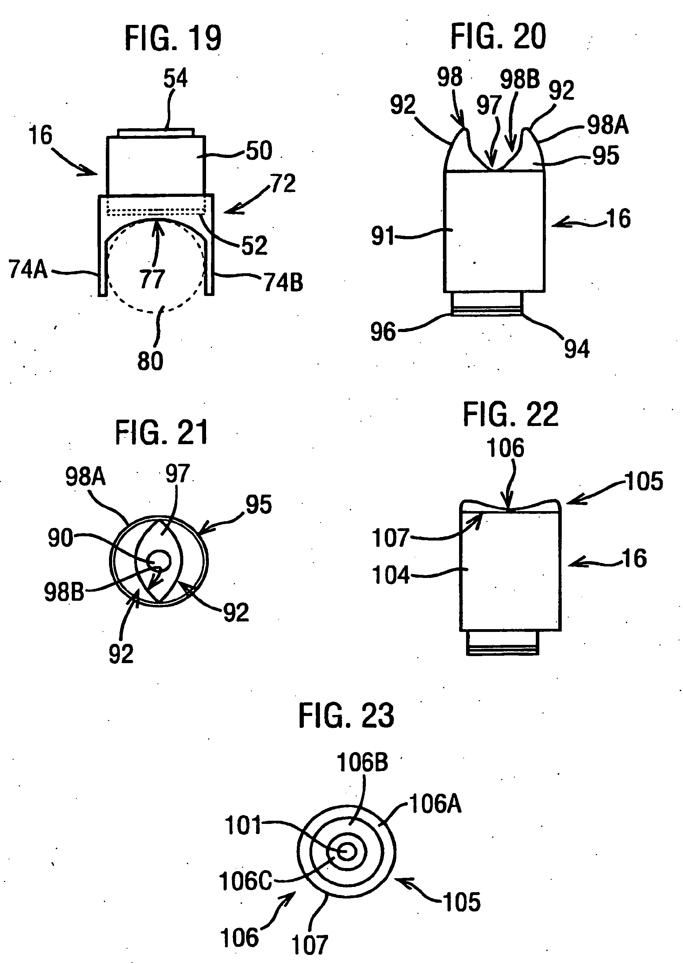

Referring to Figure 19, the legs 74A and 74B can be spaced apart a distance sufficient to allow a finger 80 of the user to fit between the legs 74A and 74B. The surface 77 connecting the two legs 74A and 74B can be curved, and are preferably parabolic in shape, to further fitting of the user's finger 80. In addition, the legs 74A and 74B, as well as the sleeve 70, can be constructed from a flexible, resilient material, such as a flexible plastic. The preferred material of choice is ABS plastic. As illustrated in Figure 19, the user's finger 80 can be positioned between the legs 74A and 74B when the sleeve 70 is positioned beneath the cap 16. The legs 74A and 74B compress the user's finger therebetween to pinch or squeeze the dermal tissue. The user's finger can then be lanced and the compression of the user's finger by the legs 74A and 74B can facilitate the expression of blood from the lancing site.

Alternate embodiments of the cap are illustrated in Figures 20 to 23, in which the contact ring 52 is designed for lancing the sharp curve (or side) of the fingertip, as well as the ventral side of the fingertip.

Figures 20 and 21 illustrate another embodiment of the lancet cap suitable for use with the present invention. The cap 16 includes a cap body 91 having a proximal end 96. A contact ring 95 is attached to the distal end 93 of the cap body 91. An opening 90 is provided in the contact ring 95 to allow a portion of the lancet 13 to pass through to effect puncturing of the fingertip. The illustrated cap body 91 can include a connector 94 for removably and replaceably connecting the proximal end 96 of the cap body 91 to the distal end of the housing 11 of the device 10 For example, the connector 94 can be sized and shaped to fit the housing 11. The lancet cap 16 can be permanently affixed to the housing 11 although it is preferable that the lancet cap 16 be removably and replaceably connected to the housing 11.

The contact ring 95 preferably employs a pair of pressure wings 92 sized and dimensioned to accommodate the sharp curve of the fingertip therebetween. The pressure wings 92 thus form a recess 97 for accommodating the finger of the user. This applies the correct amount of pressure to allow for the expression of blood.

Referring to Figure 21, pressure wings extend radially outward and away from the contact ring for contacting the fingertip both during lancing and during blood sample expression. The pressure wings 92 constitute a multi-contoured surface that extends from the outer periphery of the body 91 to the opening 102. The multi-contoured surface 98 is designed to pressure the fingertip to maximize blood flow rate from the lancing site and to facilitate the expression of blood for sample collection. The illustrated multi-contoured surface 98 comprises two or more non-planar surfaces disposed at distinct angles relative to each other and with respect to a common axis. For example, the pressure wings 92 that constitute the multi-contoured surface 98 is comprised of a radial outer portion 98A and a curved radial inner portion 98B proximate to the opening 102. The transition point between the surfaces 98A and 98B can be arcuate, rounded, or sharp.

When in use, the lancet cap 16 illustrated in Figures 20 and 21 is connected to the housing 11 of the meter 10 of the present invention, and the fingertip of the user is placed in the recess 97 formed by the pressure wings 92. The lancet 13 of the device is deployed and passes through the opening 102 in the contact ring 95 to pierce the skin. The contact ring 95 is pressed into contact with the fingertip proximate to the lancing site to express blood. The multi-contoured surface facilitates blood expression by creating a pressure gradient that extends radially inwardly towards the opening 102.

Figures 22 and 23 illustrate another embodiment of the lancet cap of the integrated blood sampling and testing meter according to the teachings of the present invention. As illustrated in Figure 22, the lancet cap 16 includes a contact ring 105 attached to the distal end 107 of the cap body 104. An opening 101 formed in the contact ring 105 allows a portion of the lancet 13 to pass therethrough to create a puncture on the ventral side of the fingertip.

The illustrated contact ring 105 has a multi-contoured surface 106 that extends from the periphery of the cap body 104 to the central opening 101. The multi-contoured surface 106 can include two or more surfaces disposed at distinct angles relative to each other and with respect to a common axis. For example, the illustrated multi-contoured surface 106 is comprised of an outer radial portion 106A, a middle portion 106B, and an inner radial portion 106C disposed proximate to the opening 101. The outer, middle and inner radial portions of the cap can have any selected surface feature or shape, e.g., can be linear, stepped, or curved. Moreover, the transition points between each surface 106A, 106B and 106C of the multi-contoured surface can have rounded, arcuate, or sharp surface features.

When in use, the lancet cap 16 is connected to the housing 11 of the meter 10 of the present invention and is placed in intimate facing contact with the ventral side of the finger which is lanced by the lancet 13 passing through the opening 101 in the contact ring 105. The lancet 13 is withdrawn into meter 10. The fingertip is squeezed into contact with the outer radial portion 106A, middle radial portion 106B, and inner radial portion 106C of the multi-contoured surface 106. The multi-contourrsurface 106. facilitates blood expression by creating a pressure gradient that extends radially inwardly toward the opening 101 from the perimeter of the contact ring 105 or cap body 104.

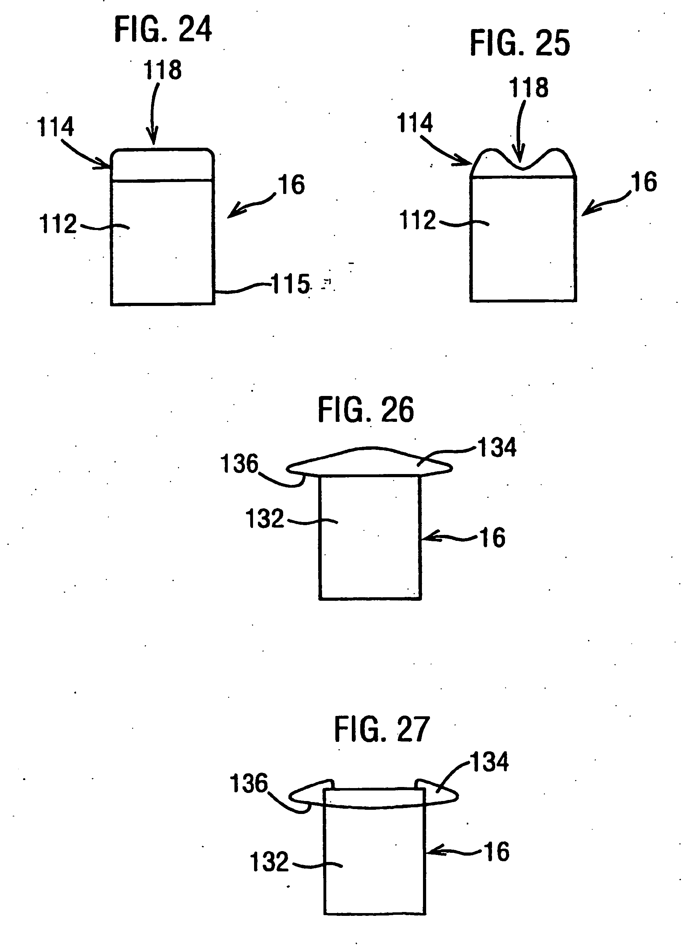

Figures 24 and 25 illustrate another embodiment of the lancet cap for use at multiple different lancing sites according to the teachings of the present invention. The illustrated lancet cap 16 includes a cap body 112 that terminates at a contact ring 114 mounted at a distal end. The distal end 115 of the cap 16 can couple to the housing 11 via any suitable structure. The contact ring 114 of the cap 16 can include, if desired, a multi-contoured surface 118 having a plurality of surfaces oriented at angles relative to each other. A central opening can also be formed therein. According to an alternate embodiment, the contact ring can be a unitary structure with nominal surface features formed therein.

The illustrated contact ring 114 is preferably formed of a deformable, resilient, flexible material that is capable of conforming to the shape of the body region of the user placed in contact therewith. The contact ring can be preferably formed of a rubber material, polyurethane, latex or other flexible material. The cap body 112 can also be formed of any suitable transparent or translucent material, such as clear or transparent plastic, to enable the user to view the expressed blood. Alternatively, the cap can be formed of a non-transparent material.

The contact ring 114 can be disposed in a rest position, shown in Figure 24, when not in contact with a lancing site, and hence no shape is imparted to the ring. When placed in contact with the lancing site, such as the ventral side of a finger, or any other suitable portion of the finger, the contact ring conforms to the shape of the lancing site, as illustrated in Figure 25.

According to an alternate embodiment, as illustrated in Figures 26 and 27, the lancet cap 16 of the integrated testing meter of the present invention can include a cap body 132 that has mounted thereto a deformable contact ring 134. The illustrated contact ring 134 has edge portions 136 that extend over or outwardly from the perimeter of the cap body 132. The contact ring can include, if desired, a multi-contoured surface having a plurality of surfaces oriented at angles relative to each other. A central opening can also be formed therein. According to an alternate embodiment, the contact ring can be a unitary structure with nominal surface features formed therein.

The contact ring can be preferably formed of rubber material, polyurethane, latex or other flexible material. The contact ring 134 can be disposed in a rest position, shown in Figure 26, when not disposed in contact with a lancing site, and hence no shape is imparted to the ring. When placed in contact with the lancing site, such as the ventral side of a finger, or any other suitable portion of the finger, the contact ring 134 conforms to the shape of the lancing site, as illustrated in Figure 27. Moreover, when disposed in this position, the overhanging portions of the deformable contact ring, can 'flip' over and extend along the outer surface of the cap body 132 so it can be used on a flatter skin area, such as the forearm.

The cap body 132 can include a connector for removably and replaceably connecting a proximal end of the cap body 132 to the housing 11. According to one practice, the lancet cap 16 can be permanently affixed to the housing 11. Preferably, the lancet cap 16 is removably and replaceably connected to the housing 11 of the integrated testing meter 10.

The lancet cap 16 for use with the integrated sampling and testing meter of the present invention is not limited to the illustrative embodiments described above. Those skilled in the art will recognize that a certain changes may be made to the lancet cap construction without departing from the scope of the invention.

A significant feature of the integrated meter of the present invention is that the strip dispensing system automatically and accurately positions the sample-receiving area of a test strip in close proximity to the puncture site formed by the lancet in order to provide a sufficient blood sample with a minimal puncture wound. Puncturing very accurately and in a defined proximity to the sample-receiving area of the test strip eliminates transfer of the sample by the user. The test strip wicks the blood sample directly from the puncture, allowing the use of low volume blood samples for analysis. Moreover, the test strip is positioned to automatically and efficiently direct the sample to a defined area of the test strip for analysis. The strip dispensing system and the feeding channel co-operate with the lancet assembly to position the sample-receiving area of the strip about one millimeter away from the puncture site. The precisely controlled distance between the puncture and the sample-receiving area of the test strip defines a minimal drop size required to be formed for analysis. When the drop of blood formed by the puncture grows to about 0.8 millimeter radius (about one microliter in volume), the drop touches the sample-receiving area of the test strip. The test strip provides a capillary force to wick the blood drop into the test strip. The described arrangement efficiently conveys a sample from the skin to a precise position on the test strip with little or no loss.

According to one practice of the invention, the test strip is flexible to accommodate deep and superficial bulging of the skin formed by the squeezing of the skin by the pressure ring in the lancet cap. According to an alternate embodiment, the test strip is precisely positioned above the puncture site, in the lancet path, so that the lancet first pierces the wicking element of the test strip, then pierces the skin.

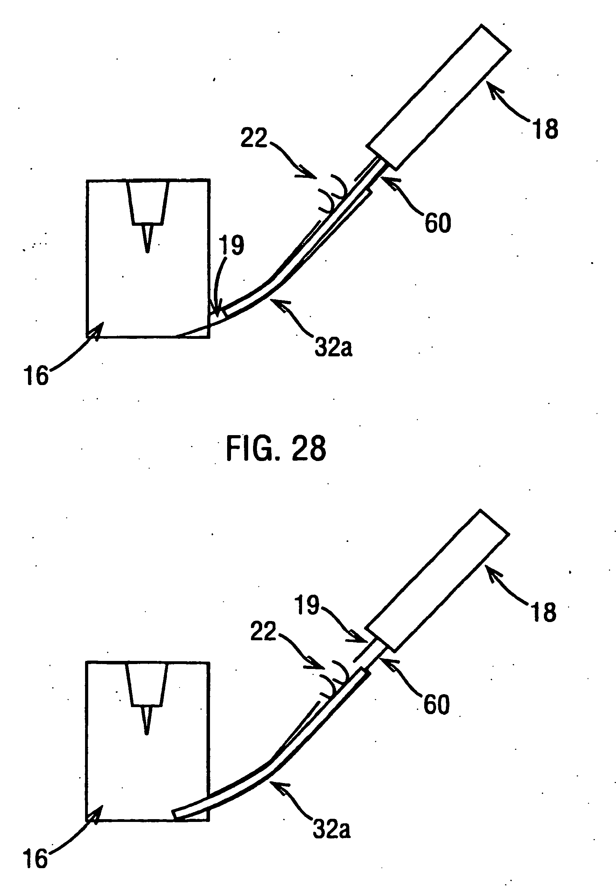

To accurately position a test strip, the feeding channel 19 of the integrated sampling and testing device of the present invention includes a step 60, illustrated in Figure 28. When the test strip 32a exits the test strip cartridge 18, and passes through the feeding channel to the sample-receiving position in the lancet cap 16, the step 60 locks the strip and prevents the strip from reversing and moving back into the test strip cartridge 18. The walls of the feeding channel further provides lateral alignment of the strip. According to the illustrated embodiment, the electrical contacts 22 connected to the electronics in the device may be spring biased or otherwise arranged to lock the test strip into place behind the step.

The cartridge cap may also include a strip ejection ramp to push the test strip out of the feeding channel when the cap is closed. The strip ejection ramp may be integral with the cartridge cap or removably connected to the cartridge cap.

The design of the lancet needle and the lancet assembly provides an accurate puncture site with little variation to ensure a precise relationship between the sample-receiving area of the test strip and the puncture site. According to one practice of the present invention, the lancet is ground to center the point on the tip of the lancet. According to an alternate embodiment, the lancet needle is ground at the edge, as with current lancets. The lancet is then positioned in plastic, such that the pointed tip is located in a fixed position in the center of the plastic. Thus, the puncture site is precisely fixed, independent of the orientation of the drive train, and/or vibrations of the drive train. The lancet cap further includes an alignment feature to control wobbling or vibration on the front end of the drive train. According to an illustrative embodiment, the lancet cap includes a plurality of alignment fins (not shown) disposed about the base of the lancet to secure the lancet. In this manner, the total variability of the puncture site is minimized.

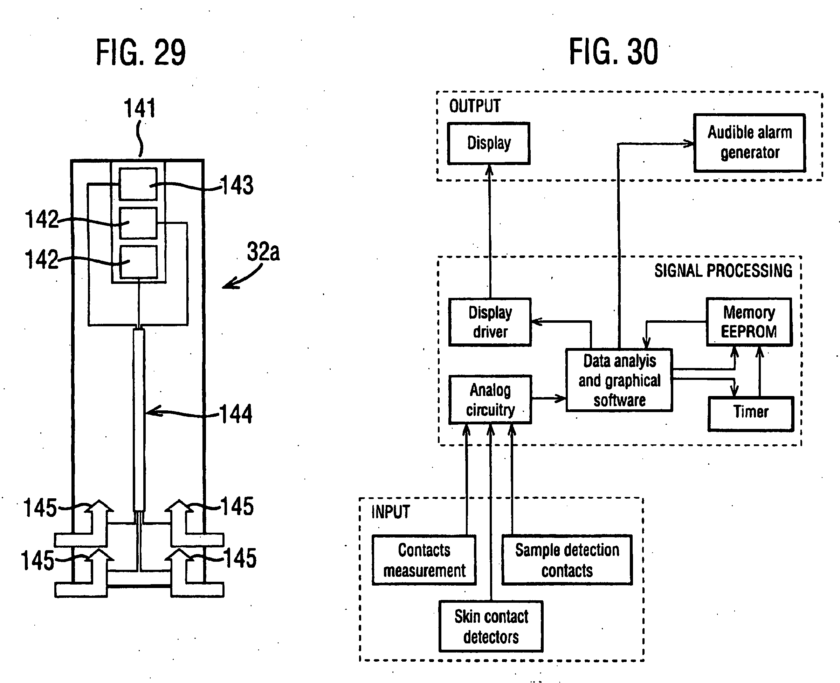

Figure 29 illustrates a test strip design suitable for use in the present invention. The test strip may utilize A-strip technology, membrane strip technology or other test strip designs known in the art for electrochemical or photometric analysis of a fluid. According to one embodiment, the test strip 32a includes, as its sample-receiving area, a channel entrance 141 for directing a blood sample to an analysis portion of the strip. The test strip essentially comprises an electrochemical cell, including one or more working electrodes 142 which convert a chemical change produced by a reaction of glucose or other analyte in the blood sample to a current. The test strip further includes a reference electrode 143 as a standard to measure the potential of the working electrodes. Leads 144 connect the electrodes to contact bars 145 configured to connect with the electrical contacts 22 of the integrated testing meter. The test strip thus generates a signal indicative of the level of glucose or other analyte in the blood and transmits this signal to the electronics of the device for processing. Those skilled in the art will recognize that a variety of test strip designs and configurations are available in accordance with the teachings of the present invention.

Figure 30 shows a schematic representation of the electronics incorporated in the integrated meter of the present invention. The electronics receive a signal from the electrical contacts, process the signal and transmit instructions for an appropriate display to the display of the device. As shown, input signals related to the electrochemical analysis of the sample are provided from the test strip to a signal processing system. The signals are transmitted via analog circuitry to a processor, which performs data analysis. The processor provides a signal to a display driver connected to an output display. The processor may also provide a signal to an alarm generator. The display and the alarm generator together constitute the output portion of the device. The data analysis processor also communicates with a memory module, such as an EEPROM, in which information, including calibration information and previous test results, may be stored.

According to one practice of the invention, the electronics further include a detector for sensing a strip in the feeding channel. The detector can be two contacts which are shorted by a conductive layer on a strip when the strip is in the sample-receiving position. The electronics may be designed to produce an audible beep or visible signal to indicate to the user that a sufficient sample has been obtained and that analysis is complete. The electronics may also read, store and/or display information regarding the date and time of testing, the condition of the strips, the number of strips remaining in the stack, a calibration code for the strips, the expiration date of the test strip cartridge, the battery power of the meter, and so on. As noted above, test strip specific information can be read directly from the cartridge, for instance by use of bar codes, resistance bridges or memory modules, preferably rewritable memory modules.

As discussed, according to one embodiment, the electronics are switched on when a user depresses the handle of the integrated testing meter, or when a test strip detector detects a loaded test strip in the sample-receiving position. Preferably, each time the electronics are switched on, the data on the cartridge is read to ensure that the correct calibration code and other data are used to control the meter. This ensures that a correct test result can be obtained even if the cartridge has been changed.

According to another embodiment of the invention, the electronics are switched off when the user replaces the test strip cartridge cap and ejects the used test strip from the meter. This provides an extra safety feature as it ensures that the cartridge remains closed for as long as possible. This minimises the exposure of the contents of the cartridge to the atmosphere. Preferably, the electronics in the meter are arranged to record the length of time between a strip reaching the sample-receiving position and its being ejected from the meter. This is a measure of the time the cap is open. If the total time the cap is open exceeds a predetermined value, the electronics may be arranged to provide an audible or visible warning signal. The electronics may also be arranged to provide such a signal, or to switch off the meter, if any single strip has remained in the sample-receiving position for longer than a predetermined time.

The integrated meter of the present invention and its components provide significant improvements to the detection and monitoring of glucose levels in the blood. The present invention considerably reduces the pain and inconvenience associated with glucose monitoring. The invention further improves the efficiency and accuracy of testing by providing an automated transfer and analysis of the sample. The invention provides an integrated testing meter with user-friendly, uncomplicated operation. The integrated testing meter is compact, ergonomically sound, discrete and adjustable to different users and body parts while simultaneously providing fast and accurate results.

The present invention achieves a reduction in the pain associated with testing in a number of ways. Shallower punctures of the skin can be used to achieve a sufficient blood sample, reducing painful deep punctures in sensitive body parts. The present invention does not require large sample volumes for analysis. The pressure device, for instance formed by the pressure ring on the lancet cap, provides a high yield from a small puncture. The integrated sampling and testing feature further ensures full usage of the obtained sample and limits "leftovers" on the skin. In current systems, complex and inaccurate sample transfer from a sampling point to a sample-receiving area on a test strip requires surplus sample due to poor utilization of an obtained sample drop. The present invention removes this inefficiency of transferring samples and provides optimal utilization of the obtained sample by automatically directing the sample to a precise location on the test strip. Optimal utilization of the sample drop reduces the number of attempts needed to provide enough sample for efficient analysis, thus reducing the number of punctures required. The superficial punctures reduce agitation of nerve endings in the skin and reduce pain in sensitive body areas. The variable depth of the lancet and the ability to test on a number of different body parts in addition to the finger reduces the concentration and repetition of micro-traumata in a small area, which avoids the problems of tinting, itching, dried and callous skin areas caused by such micro-traumata.

The integrated meter of the present invention is able fully to exploit the technological improvements in strip design which allow the use of much smaller samples. Presently available strips require only 1 to 3 µl of sample. Many users have eyesight problems and cannot see well enough to be able to transfer such small volumes of sample accurately to the sample-receiving area of a strip. Such eyesight problems are common complications of diabetes. The automatic collection and transfer of samples to the strip enabled by the present meter is a major advantage of the present meter.

It is thus possible to arrange the operation of the lancet and the pressure device to produce a puncture which allows a small, but sufficient, volume of blood to be expressed as a drop on the user's skin. Such a small drop will be able to contact the sample-receiving area of the test strip due to the precise positioning of the lancet and the test strip.

The small volume of blood or other bodily fluid expressed from the user is sufficient to accurately determine or monitor the presence or absence of an analyte, such as glucose.

The present invention further provides easy and uncomplicated operation. The use of the meter significantly reduces the time and difficulty involved in sampling and testing blood. The integrated meter essentially provides three devices, a lancing device, a supply of test strips and a meter, within a singular compact housing. Further, the system is designed such that one-handed operation is possible, eliminating the need for a work space or a flat surface. The meter is not subject to human error and inefficiency. Furthermore, the integration of a disposable test strip cartridge makes the loading of a test strip simple, accurate and easy. In current glucose monitoring systems a user requires two hands to load a strip into a glucose meter. However, with the meter of the present invention, the test strip dispensing system automatically loads a test strip in position to receive a blood sample. The present invention also reduces waste by efficiently utilizing available resources. The present invention further protects against compromised test results due to contamination or an improperly calibrated glucose meter.

In conclusion, the integrated meter of the present invention significantly reduces the obstacles associated with frequent glucose monitoring. The present invention promotes frequent monitoring for diabetic individuals by providing a simple, efficient, fast and accurate integrated meter.

Since certain changes may be made in the above constructions without departing from the scope of the invention, it is intended that all matter contained in the above description or shown in the accompanying drawings be interpreted as illustrative and not in a limiting sense.

It is also to be understood that the following claims are to cover all generic and specific features of the invention described herein, and all statements of the scope of the invention which, as a matter of language, might be said to fall therebetween.

## Claims

1. An integrated sample-testing meter (10) comprising a single modular housing (11) carrying:
a pressure device (52);
a lancet (13);
a lancet drive train (14) for driving the lancet (13) between an extended position and a retracted position;
a test strip cartridge (18) containing a plurality of test strips (32), each strip having a sample-receiving area;
a sensor for analyzing a fluid sample received on a test strip (32); and
a test strip dispensing system (20) for moving test strips (32) individually from the cartridge (18) to a sample-receiving position in which the sample-receiving area of the test strip (32) is in proximity to the location of the end of the lancet (13) in its extended position and in which the test strip (32) is connected to the sensor,
the meter (10) being arranged such that, In use, when it is located on the skin of a user and is activated, the lancet (13) is moved to its extended position and retracted to form a puncture in the user's skin, the pressure device (52) causes a drop of fluid to form around the puncture, a test strip (32) is moved from the cartridge (18) to the sample-receiving position, the test strip (32) receives a sample from the fluid drop and the sensor analyzes the sample; and wherein
the meter (10) includes a feeding channel (19) which receives the strip (32) from the cartridge (18) and guides it to the sample-receiving position, wherein the feeding channel (19) includes a step (60) arranged such that when the strip (32) has been moved past the step (60), the strip drops, or is forced, into the step (60), thereby preventing the strip from moving back away from the sample receiving position towards the cartridge (18).

2. The meter (10) of claim 1, which is arranged such that, in use, when a strip (32) is in the sample-receiving position, its sample-receiving area is spaced from the puncture site by a distance of from about 0.4 mm to about 1.3 mm.

3. The meter (10) of claim 2, wherein the distance is from about 0.7 mm to about 0.9 mm.

4. The meter (10) of any one of claims 1 to 3, wherein the lancet drive train (14) includes an adjustment screw (17) which allows the user to set the extended position of the lancet (13).

5. The meter (10) of claim 4, wherein the operation of the adjustment screw (17) is arranged such that the distance of travel of the lancet (13) remains constant.

6. The meter (10) of any one of claims 1 to 5, wherein the test strip cartridge (18) comprises a cartridge housing (31) defining a cavity configured to receive a stack of test strips (32), a partially detachable cartridge cap (33) and a means for moving the stack of test strips (32) towards the cartridge cap (33).

7. The meter (10) of claim 6, wherein the cartridge (18) is manufactured from a material having dessicant properties.

8. The meter (10) of any one of claims 1 to 7, wherein the strip (32) is forced into the step (60) by springs located on the meter (10).

9. The meter (10) of claim 8, wherein the springs are electrically conductive and are arranged to make electrical contact with electrodes or a conductive bar on the strip (32).

10. The meter (10) of any one of claims 1 to 9, wherein the feeding channel (19) is arranged such that the major plane of the strip (32), when in the sample-receiving position, is at an angle of from about 30° to about 60° to the direction of movement of the lancet towards its extended position.

## Patentansprüche

1. Integriertes Proben-Prüfmessgerät (10), das ein einziges modulares Gehäuse (11) aufweist, welches:
eine Druckeinheit (52);
eine Lanzette (13);
einen Lanzettenantriebszug (14) zum Treiben der Lanzette (13) zwischen einer ausgefahrenen Position und einer zurückgezogenen Position;
ein Teststreifenmagazin (18), das eine Vielzahl von Teststreifen (32) enthält, wobei jeder Streifen eine Proben aufnehmende Fläche hat;
einen Sensor zum Analysieren einer Fluidprobe, die auf einem Teststreifen (32) aufgenommen ist; und
ein Teststreifen-Ausgabesystem (20) zum einzelnen Bewegen von Teststreifen (32) aus dem Magazin (18) zu einer Proben aufnehmenden Position, in der sich der Proben aufnehmende Bereich auf dem Teststreifen (32) in der Nähe des Ortes des Endes der Lanzette (13) in ihrer ausgefahrenen Position befindet und in der der Teststreifen (32) mit dem Sensor verbunden ist,
enthält;
wobei das Messgerät (10) so angeordnet ist, dass, im Einsatz, wenn es sich auf der Haut eines Benutzers befindet und aktiviert ist, die Lanzette (13) in ihre ausgefahrene Position bewegt und zurückgezogen wird, um eine Punktur in der Haut des Benutzers zu bilden, wobei die Druckeinheit (52) bewirkt, dass sich ein Tropfen Fluid um die Punktur bildet, wobei ein Teststreifen (32) aus dem Magazin (18) in die Proben aufnehmende Position bewegt wird, wobei der Teststreifen (32) eine Probe von dem Fluidtropfen aufnimmt und der Sensor die Probe analysiert; und wobei
das Messgerät (10) einen Zuführkanal (19) umfasst, der den Streifen (32) aus der Kassette (18) aufnimmt und ihn in die Proben aufnehmende Position führt, wobei der Zuführkanal (19) eine Stufe (60) umfasst, die so angeordnet ist, dass, wenn sich der Streifen (32) über die Stufe (16) bewegt hat, der Streifen in die Stufe (60) abfällt oder dazu gezwungen wird, so dass verhindert wird, dass sich der Streifen zurück, weg von der Proben aufnehmenden Position, auf das Magazin (18) zu bewegt.

2. Messgerät (10) nach Anspruch 1, das derart angeordnet ist, dass, im Einsatz, wenn ein Streifen (32) in der Proben aufnehmenden Position ist, seine Proben aufnehmende Fläche von dem Ort der Punktur in einer Entfernung von ungefähr 0,4 mm bis ungefähr 1,3 mm beabstandet ist.

3. Messgerät (10) nach Anspruch 1, bei dem die Entfernung ungefähr 0,7 mm bis ungefähr 0,9 mm beträgt.

4. Messgerät (10) nach einem der Ansprüche 1 bis 3, bei dem der Lanzettenantriebszug (14) eine Einstellschraube (17) umfasst, die es dem Benutzer erlaubt, die ausgefahrene Position der Lanzette (13) einzustellen.

5. Messgerät (10) nach Anspruch 4, bei dem die Betätigung der Einstellschraube (17) derart eingerichtet ist, dass der Bewegungsweg der Lanzette (13) konstant bleibt.

6. Messgerät (10) nach einem der Ansprüche 1 bis 5, bei dem das Teststreifenmagazin (18) ein Magazingehäuse (31), welches einen Hohlraum definiert, der so gestaltet ist, dass er einen Stapel Teststreifen (32) aufnimmt, eine teilweise lösbare Magazinkappe (33) und eine Einrichtung zum Bewegen des Stapels der Teststreifen (32) auf die Magazinkappe (33) zu aufweist.

7. Messgerät (10) nach Anspruch 6, bei dem die Kassette (18) aus einem Material mit Trockeneigenschaften hergestellt ist.

8. Messgerät (10) nach einem der Ansprüche 1 bis 7, bei dem der Streifen (32) durch Federn, die sich auf dem Messgerät (10) befinden, in die Stufe (60) gezwungen wird.

9. Messgerät (10) nach Anspruch 8, bei dem die Federn elektrisch leitend sind und so angeordnet sind, dass sie einen elektrischen Kontakt mit Elektroden oder einer leitenden Leiste auf dem Streifen (32) herstellen.

10. Messgerät (10) nach einem der Ansprüche 1 bis 9, bei dem der Zuführkanal (19) derart angeordnet ist, dass die Hauptebene des Streifens (32), wenn er in der Proben aufnehmenden Position ist, sich unter einem Winkel von ungefähr 30° bis ungefähr 60° zu der Bewegungsrichtung der Lanzette in ihre ausgefahrene Position befindet.

## Revendications

1. Appareil d'analyse d'échantillons intégré (10) comprenant un seul boîtier modulaire (11) doté :
d'un dispositif de pression (52) ;
d'une lancette (13) ;
d'une transmission de lancette (14) destinée à faire passer la lancette (13) d'une position déployée à une position rétractée ;
d'une cartouche de bandelettes réactives (18) contenant une pluralité de bandelettes réactives (32), chaque bandelette ayant une zone de réception d'échantillon ;
d'un capteur destiné à analyser un échantillon de fluide reçu sur une bandelette réactive (32) ; et
d'un système de distribution de bandelettes réactives (20) destiné à déplacer les bandelettes réactives (32) individuellement de la cartouche (18) à une position de réception d'échantillon dans laquelle la zone de réception d'échantillon de la bandelette réactive (32) se trouve à proximité de l'emplacement de l'extrémité de la lancette (13) dans sa position déployée et dans laquelle la bandelette réactive (32) est connectée au capteur,
l'appareil d'analyse (10) étant agencé de sorte que, durant l'utilisation, lorsqu'il se trouve sur la peau d'un utilisateur et est activé, la lancette (13) est déplacée dans sa position déployée puis rétractée pour faire une piqûre dans la peau de l'utilisateur, le dispositif de pression (52) entraîne la formation d'une goutte de fluide autour de la piqûre, une bandelette réactive (32) est déplacée de la cartouche (18) à la position de réception d'échantillon, la bandelette réactive (32) reçoit un échantillon de la goutte de fluide et le capteur analyse l'échantillon ; et dans lequel
l'appareil d'analyse (10) comprend un canal d'alimentation (19) qui reçoit la bandelette (32) de la cartouche (18) et la guide jusqu'à la position de réception d'échantillon, le canal d'alimentation (19) comprenant un renfoncement (60) agencé de manière à ce que, lorsque la bandelette (32) a été déplacée au-delà du renfoncement (60), la bandelette tombe, ou soit contrainte de pénétrer, dans le renfoncement (60), empêchant ainsi la bandelette de repartir en arrière dans la direction de la cartouche (18).

2. Appareil d'analyse (10) selon la revendication 1, qui est agencé de manière à ce que, durant l'utilisation, lorsqu'une bandelette (32) se trouve dans la position de réception d'échantillon, sa zone de réception d'échantillon soit espacée du site de piqûre par une distance d'environ 0,4 mm à environ 1,3 mm.

3. Appareil d'analyse (10) selon la revendication 2, dans lequel la distance est d'environ 0,7 mm à environ 0,9 mm.

4. Appareil d'analyse (10) selon l'une quelconque des revendications 1 à 3, dans lequel la transmission de lancette (14) comprend une vis de réglage (17) qui permet à l'utilisateur de définir la position déployée de la lancette (13).

5. Appareil d'analyse (10) selon la revendication 4, dans lequel le maniement de la vis de réglage (17) est destiné à maintenir constante la distance de déplacement de la lancette (13).

6. Appareil d'analyse (10) selon l'une quelconque des revendications 1 à 5, dans lequel la cartouche de bandelettes réactives (18) comprend un boîtier de cartouche (31) définissant une cavité configurée pour recevoir une pile de bandelettes réactives (32), un capuchon de cartouche partiellement détachable (33) et des moyens permettant de déplacer la pile de bandelettes réactives (32) vers le capuchon de cartouche (33).

7. Appareil d'analyse (10) selon la revendication 6, dans lequel la cartouche (18) est fabriquée à partir d'un matériau ayant des propriétés desséchantes.

8. Appareil d'analyse (10) selon l'une quelconque des revendications 1 à 7, dans lequel la bandelette (32) est contrainte de pénétrer dans le renfoncement (60) par des ressorts situés sur l'appareil d'analyse (10).

9. Appareil d'analyse (10) selon la revendication 8, dans lequel les ressorts sont électroconducteurs et sont agencés pour établir un contact électrique avec des électrodes ou une barre conductrice sur la bandelette (32).

10. Appareil de mesure (10) selon l'une quelconque des revendications 1 à 9, dans lequel le canal d'alimentation (19) est agencé de manière à ce que le plan principal de la bandelette (32), dans la position de réception d'échantillon, se trouve à un angle d'environ 30° à environ 60° avec la direction de mouvement de la lancette dans sa position déployée.
